# EUROPÄISCHE PATENTSCHRIFT

(11) **EP 2 434 748 B1**
(45) Veröffentlichungstag und Bekanntmachung des Hinweises auf die Patenterteilung: **20.11.2013**
(21) Anmeldenummer: 11182213.6
(22) Anmeldetag: 21.09.2011
(51) Int. Cl.: H04N 5/225, A61B 1/05

(54) **Bildaufnehmermodul sowie Verfahren zur Herstellung eines Bildaufnehmermoduls**
Image capturing module and method for manufacturing the same
Module d'enregistrement d'image et procédé de fabrication d'un module d'enregistrement d'image

(30) Priorität: 27.09.2010 DE 102010047288
(43) Veröffentlichungstag der Anmeldung: 28.03.2012
(73) Patentinhaber: Karl Storz GmbH & Co. KG, 78532 Tuttlingen (DE)
(72) Erfinder: Eismann, Dirk, 2514 Ligerz (CH); Klumpp, Martin, 78532 Tuttlingen (DE); Schwarz, Peter, 78532 Tuttlingen-Nendingen (DE)
(74) Vertreter: Witte, Weller & Partner

(56) Entgegenhaltungen:
- US-A- 5 021 888
- US-A- 5 879 285
- US-A1- 2010 041 268

## Beschreibung

Die Erfindung betrifft ein Bildaufnehmermodul, insbesondere für ein Endoskop, mit einem elektronischen Bildsensor, der eine Mehrzahl an Kontaktfingern aufweist, die in zumindest einer Reihe angeordnet sind; einer ersten Platine, die zumindest ein elektronisches oder elektrisches Bauelement aufweist, wobei die erste Platine parallel zu einer Lichtempfangsfläche des Bildsensors angeordnet ist; einer zweiten Platine, die auf einer dem Bildsensor abgewandten Seite der ersten Platine parallel zur ersten Platine angeordnet ist, wobei die zweite Platine eine Mehrzahl an Bohrungen aufweist, die von einer der ersten Platine abgewandten zu einer der ersten Platine zugewandten Oberfläche der zweiten Platine durchgehend und flächig über die zweite Platine zwischen Rändern der zweiten Platine verteilt sind; einem Kabel, das eine Mehrzahl von Adern aufweist, mit der zweiten Platine verbunden ist und von der der ersten Platine abgewandten Oberfläche der zweiten Platine wegführt, wobei die Adern in die Bohrungen der zweiten Platine eingeführt sind, und Enden der Adern sich etwa an der der ersten Platine zugewandten Oberfläche der zweiten Platine befinden, gemäß dem Oberbegriff des Anspruchs 1.

Die Erfindung betrifft ferner ein Verfahren zum Herstellen eines Bildaufnehmermoduls.

Ein Bildaufnehmermodul der eingangs genannten Art ist aus US 5 879 285 A bekannt.

Ein elektronisches Bildaufnehmermodul der eingangs genannten Art wird vorzugsweise in einem Endoskop, insbesondere in einem flexiblen Endoskop verwendet, wobei das Bildaufnehmermodul im distalen Ende des Endoskopschafts angeordnet wird. Ein solches Endoskop bzw. Videoendoskop ist beispielsweise in US 5,754,313 beschrieben. Andere beispielhafte Verwendungen des erfindungsgemäßen Bildaufnehmermoduls sind der Einbau in ein Mikroskop, eine medizinische Miniaturkamera, ein Exoskop, etc.)

Ein Bildaufnehmermodul umfasst allgemein einen elektronischen Bildsensor, der auf ihn einfallendes Licht in elektrische Signale umwandelt. Allgemein sind solche elektronischen Bildsensoren in CCD- oder CMOS-Technologie ausgeführt.

Miniaturisierte Bildsensoren sind derzeit erhältlich, unter denen solche bevorzugt werden, die in TAB (Tape Automated Bonding)-Technologie gefertigt sind. Derartige Bildsensoren weisen in zumindest einer Reihe, üblicherweise in zwei Reihen an gegenüberliegenden Schmalseiten des Bildsensors angeordnete Kontaktfinger auf, die sich beim Bildaufnehmermodul etwa senkrecht zur Lichtempfangsseite des Bildsensors von diesem weg erstrecken.

Die Verwendung eines Bildaufnehmermoduls für ein Endoskop, bei dem das Bildaufnehmermodul in die distale Spitze des Endoskopschafts eingebaut wird, bedingt eine miniaturisierte Baugröße des Bildaufnehmermoduls. Die Herstellung eines miniaturisierten Bildaufnehmermoduls ist jedoch mit technischen Problemen behaftet, insbesondere was die Assemblierung der Anordnung aus Bildsensor, Platine und Kabel angeht.

Im Stand der Technik sind verschiedene Bauweisen von Bildaufnehmermodulen beschrieben worden, die jedoch die technischen Probleme bei der Assemblierung der Komponenten des Bildaufnehmermoduls nicht zufriedenstellend lösen.

In dem Dokument EP 2 018 043 A1 ist in den dortigen Figuren 4a und 4b ein Bildaufnehmermodul gezeigt, das zwei Platinen aufweist, die in Richtung senkrecht zur Lichtempfangsfläche des Bildsensors hintereinander angeordnet sind. Die vom Bildsensor aus gesehen erste Platine ist dabei mit elektronischen oder elektrischen Bauelementen bestückt. Die zweite Platine, die auf der dem Bildsensor abgewandten Seite der ersten Platine angeordnet ist, dient der Terminierung des mehradrigen Kabels. Dazu weist die zweite Platine eine Mehrzahl an durchgehenden Bohrungen auf, in die die einzelnen Adern des Kabels eingeführt sind. Die Enden der Adern befinden sich dabei etwa an der inneren Oberfläche der insgesamt U-förmig ausgebildeten zweiten Platine. Im assemblierten Zustand umgreifen die Kontaktfinger des Bildsensors die zweite Platine auf deren Rückseite, um die Kontaktfinger mit der zweiten Platine elektrisch leitend zu kontaktieren. Aufgrund der U-förmigen Ausgestaltung der zweiten Platine ist die zweite Platine mit der ersten Platine über Drähte elektrisch leitend kontaktiert, was sich jedoch bei einer miniaturisierten Ausgestaltung der beiden Platinen schwierig gestaltet, und sich insbesondere nicht für eine automatisierte Assemblierung mittels eine Roboters eignet.

In dem vorstehend genannten Dokument ist in einem weiteren Ausführungsbeispiel in den dortigen Fig. 7a bis 7c ein Bildaufnehmermodul gezeigt, das nur eine einzige Platine aufweist. Diese Platine weist durchgehende Bohrungen auf, durch die die Adern des Kabels hindurchgeführt sind, wobei die Adern mit ihren Adermänteln aus der dem Bildsensor zugewandten Oberfläche der Platine herausgeführt und umgebogen sind, um die Enden der Aderseelen nach ihrer Abisolierung mit der Platine elektrisch leitend zu kontaktieren. Bei dieser Ausgestaltung ergibt sich das Problem, dass die Enden der Adern abisoliert werden müssen, damit die Aderseelen auf die dem Bildsensor zugewandte Oberfläche der Platine aufgelötet werden können. Aus diesem Grund eignet sich auch dieses Bildaufnehmermodul nicht für eine automatisierte Assemblierung mittels eines Roboters.

Aus dem Dokument EP 1 659 780 A1 ist ein weiteres Bildaufnehmermodul bekannt, das eine einzige Platine aufweist, die sowohl mit elektrischen oder elektronischen Bauteilen bestückt ist und der Terminierung des mehradrigen Kabels dient. Die Platine ist U-förmig oder mit einer Einkerbung in der dem Bildsensor zugewandten Oberfläche ausgebildet. Das zumindest eine elektronische Bauelement ist bei diesem Bildaufnehmermodul mittels Leiterdrähten elektrisch leitend mit der Platine kontaktiert, die in der durch die U-förmige Ausgestaltung bzw. durch die Einkerbung geschaffenen Raum dreidimensional verlegt sind. Eine solche "Luftverdrahtung" der Komponenten des Bildaufnehmermoduls eignet sich ebenfalls nicht für eine automatisierte Herstellung, insbesondere nicht für eine automatisierte Herstellung mittels eines Roboters. Die Adern des Kabels sind bei diesem bekannten Bildaufnehmermodul an der Rückseite der Platine kontaktiert.

In dem Dokument WO 00/72744 A2 ist ein Bildaufnehmermodul beschrieben, dessen Platine aus einer entlang flexibler Verbindungsabschnitte faltbaren einteiligen Platte gebildet ist, die sich zu einem quaderförmigen, im Querschnitt im Wesentlichen U-förmigen Körper falten lässt. Der Platinenkörper weist im gefalteten Zustand zwei sich im Wesentlichen quer zum Bildsensor erstreckende und voneinander beabstandete Abschnitte und einen dritten Abstand auf, der im Wesentlichen parallel zum Bildsensor verläuft. Auch ein solches Bildaufnehmermodul eignet sich nicht für eine automatisierte Assemblierung der einzelnen Komponenten des Bildaufnehmermoduls.

Aus dem Dokument US 6 494 739 B1 ist ein Bildaufnehmermodul bekannt, das ein Verbindungsstück aufweist, mit dem der Bildsensor und eine Steuerplatine verbunden sind, und das zur elektrischen Kontaktierung eines mehradrigen Kabels dient. Die Adern des mehradrigen Kabels sind in seitlichen Vertiefungen in Form von Nuten, die metallisiert sind, an dem Verbindungsstück festgelegt und mit den Metallisierungen kontaktiert. Die Steuerplatine mit dem Bildsensor ist ebenfalls über Metallisierungen mit dem Verbindungsstück elektrisch kontaktiert. In einem weiteren Ausführungsbeispiel in diesem Dokument ist das Verbindungsstück aus zwei planparallelen Platten aufgebaut, wobei die dem Bildsensor abgewandte Platte der elektrischen Kontaktierung der Abschirmungen der einzelnen Adern des mehradrigen Kabels und die dem Bildsensor zugewandte Platte der Kontaktierung der Signalleiter der Adern des mehradrigen Kabels dient. Auch hier sind an den beiden Platten seitlich, das heißt an deren Schmalseiten, nutartige Vertiefungen zur Aufnahme der Abschirmungen bzw. der Leiter vorhanden. Das Verbindungsstück weist keine durchgehenden Bohrungen zum Durchführen der Adern des mehradrigen Kabels auf.

Ein weiteres Bildaufnehmermodul ist aus dem Dokument US 2004/0263680 A1 bekannt. In einer dort beschriebenen Ausführungsform sind zwei planparallele Platinenteile vorhanden, die in Längsrichtung des Bildaufnehmermoduls vergleichsweise weit voneinander beabstandet sind. Die Adern eines mehradrigen Kabels sind teilweise mit der Unterseite des vom Bildsensor abgewandten Platinenteils elektrisch kontaktiert, teilweise durch Bohrungen in diesem Platinenteil durchgeführt und als Luftverdrahtung, den Abstand zum dem Bildsensor zugewandten Platinenteil überbrückend, mit letzterem elektrisch kontaktiert.

Neben der fehlenden Eignung der bekannten Bildaufnehmermodule für eine automatisierte Assemblierung der Komponenten des Bildaufnehmermoduls haben die bekannten Bildaufnehmermodul den weiteren Nachteil, dass die Bildaufnehmermodule erst einem Funktionstest unterzogen werden können, wenn sie vollständig assembliert sind, d.h. wenn auch der Bildsensor mit der oder den Platinen elektrisch leitend kontaktiert ist. Wenn jedoch die Kontaktierungen und Verdrahtungen der Platine oder der Platinen fehlerhaft ist, ist das gesamte Bildaufnehmermodul einschließlich des teuren Bildsensors unbrauchbar. Bei den bekannten Bildaufnehmermodulen tritt somit eine unerwünscht hohe Ausschussrate von an sich ordnungsgemäß funktionierenden Bildsensoren auf.

Bei den bekannten Bildaufnehmermodulen müssen die Adern des mehradrigen Kabels vor dem Anlöten an die zumindest eine Platine einzeln abisoliert und auf die richtige Länge zugeschnitten werden. Dabei müssen die einzelnen Adern möglichst kurz hinter der Kabelwurzel enden, damit die Baulänge des Gesamtmoduls so klein wie möglich ist. Diese Anforderung erschwert das Abisolieren und Ablängen der Kabel extrem und macht diesen Vorgang ebenfalls sehr zeitaufwendig.

Das aus dem eingangs genannten Dokument US 5 879 285 A bekannte Bildaufnehmermodul weist einen Bildsensor, eine Steuerplatine und ein mehradriges Kabel auf. Das mehradrige Kabel ist über einen Block mit der Steuerplatine verbunden. Der Block weist entsprechend der Anzahl an Adern des mehradrigen Kabels eine Anzahl von Bohrungen auf, die durch den Block hindurchgehen. Die einzelnen Adern des mehradrigen Kabels werden mit ihren Adermänteln in die Bohrungen einzeln eingesetzt, bis die äußersten Enden der Adern auf der distalen Seite des Blocks zu liegen kommen. Die einzelnen Adern können dabei in den Bohrungen des Blocks mittels eines Klebstoffs fixiert sein. Überstehende Enden der Adern werden dann abgeschnitten. Die Steuerplatine weist auf ihrer dem Block zugewandten Seite Kontaktstelle auf, und zwar entsprechend der Anzahl an Adern. Zum Kontaktieren der Platine mit dem mehradrigen Kabel wird zwischen den Block und die Platine zunächst ein anisotropes leitendes Blatt eingefügt, und die Platine wird mit ihren Kontaktstellen gegen den Block gedrückt, so dass die Kontaktstellen und die Adern des Kabels miteinander elektrisch kontaktiert sind. Die Kontaktierung des Bildsensors mit der Steuerplatine erfolgt über Elektroden, die zwischen dem distalen Ende der Steuerplatine und dem proximalen Ende des Bildsensors angeordnet sind.

Der Erfindung liegt die Aufgabe zugrunde, ein Bildaufnehmermodul der eingangs genannten Art dahingehend weiterzubilden, dass es sich auf einfache Weise assemblieren lässt.

Der Erfindung liegt des Weiteren die Aufgabe zugrunde, ein Verfahren zum Herstellen eines Bildaufnehmermoduls anzugeben.

Erfindungsgemäß wird die an erster Stelle genannte Aufgabe hinsichtlich des eingangs genannten Bildaufnehmermoduls durch die kennzeichnenden Merkmale des Anspruchs 1 gelöst.

Das erfindungsgemäße Bildaufnehmermodul geht von einer Bauweise aus, bei der anstatt einer einzigen Platine zwei Platinen vorhanden sind, von denen die erste Platine mit zumindest einem elektronischen oder elektrischen Bauelement bestückt ist, während die zweite Platine, die auf der dem Bildsensor abgewandten Seite der ersten Platine angeordnet ist, der Terminierung des mehradrigen Kabels dient. Beide Platinen sind dabei parallel zur Lichtempfangsseite des Bildsensors angeordnet. Prinzipiell können beide Platinen die Form eines Quaders aufweisen, deren Hauptflächen parallel zur Lichtempfangsseite des Bildsensors angeordnet sind, und deren zu diesen Oberflächen senkrechten Oberflächen Schmalseiten der jeweiligen Platine bilden.

In der zweiten Platine sind eine Mehrzahl an Bohrungen vorhanden, die zwischen den beiden parallel zur Lichtempfangsseite des Bildsensors verlaufenden Oberflächen der zweiten Platine durchgehend sind. In diese Bohrungen sind die Adern des mehradrigen Kabels eingeführt, so dass Enden der Adern sich etwa an der der ersten Platine zugewandten Oberfläche der zweiten Platine befinden.

Im Unterschied zu der Bauweise des bekannten Bildaufnehmermoduls, das ebenfalls zwei Platinen aufweist, weist die zweite Platine des erfindungsgemäßen Bildaufnehmermoduls an ihrer der ersten Platine zugewandten Oberfläche nicht ein U-Profil oder eine Einkerbung auf, sondern die der ersten Platine zugewandte Oberfläche der zweiten Platine ist insgesamt im Wesentlichen eben. Hieraus ergibt sich der Vorteil, dass bei der Terminierung des mehradrigen Kabels an der zweiten Platine die einzelnen Adern mit ihren Adermänteln durch die Bohrungen in der zweiten Platine durchgeführt werden können, bis die Enden der Adern auf der der Kabelwurzel abgewandten Oberfläche der zweiten Platine vorstehen. Anschließend ist es dann auf einfache Weise möglich, durch Schneiden entlang dieser Oberfläche senkrecht zu den Adern die Adern bündig mit dieser Oberfläche der zweiten Platine abzulängen. Dieser Vorgang kann mit einer entsprechenden Vorrichtung automatisiert durchgeführt werden. Ein Abisolieren der Adermäntel entfällt dabei vorteilhafterweise, weil durch das Schneiden die Aderseelen stirnseitig stumpf freigelegt werden.

Bei dem erfindungsgemäßen Bildaufnehmermodul ist weiterhin die erste Platine an ihrer der zweiten Platine zugewandten Oberfläche mit einer Mehrzahl an punktförmigen ersten elektrischen Kontaktstellen versehen, deren Flächenverteilung der Flächenverteilung der Enden der Adern an der Oberfläche der zweiten Platine entspricht. Über diese punktförmigen elektrischen Kontaktstellen ist die erste Platine unmittelbar mit den Adern elektrisch leitend kontaktiert. Dies trägt ebenfalls zur vereinfachten und insbesondere für eine Automatisierung geeigneten Assemblierung der zweiten Platine mit der ersten Platine bei. Die erste Platine muss nämlich nur auf die erste Platine aufgesetzt werden, um die elektrische Kontaktierung der ersten Platine mit der zweiten Platine zu bewerkstelligen. Eine Luftverdrahtung wie bei den bekannten Bildaufnehmermodulen, die sich nicht für eine automatisierte Assemblierung eignet, entfällt somit vorteilhafterweise.

Unter "punktförmige elektrische Kontaktstellen" ist hier zu verstehen, dass die ersten Kontaktstellen in Form winziger Inseln an der der zweiten Platine zugewandten Oberfläche der ersten Platine ausgebildet sind.

Die ersten Kontaktstellen sind vorzugsweise so flach, dass sie gegenüber der Oberfläche der ersten Platine, an der sie vorhanden sind, nicht oder nicht wesentlich erhöht sind. Hierdurch ist es dann möglich, die erste Platine und die zweite Platine im Sinne einer axial kurz bauenden Bauweise sehr nahe aneinander mit geringem Spalt dazwischen anzuordnen.

Das erfindungsgemäße Bildaufnehmermodul lässt sich somit zumindest, was die Terminierung des mehradrigen Kabels und die Kontaktierung der ersten Platine mit der zweiten Platine angeht, in einem automatisierten Verfahren herstellen.

Außerdem baut das erfindungsgemäße Bildaufnehmermodul in axialer Richtung, d.h. senkrecht zur Lichtempfangsfläche des Bildsensors vorteilhafterweise sehr kurz.

Die erste Platine weist an zumindest einer Schmalseite eine Mehrzahl an zweiten Kontaktstellen auf, wobei die Kontaktfinger des Bildsensors an den zweiten Kontaktstellen elektrisch leitend kontaktiert sind.

Hierbei ist von Vorteil, dass die gegen mechanische Beanspruchung empfindlichen Kontaktfinger des Bildsensors nach dem Entnehmen des Bildsensors aus seiner Verpackung nur einmal umgebogen werden müssen, nämlich aus einer Richtung parallel zur Lichtempfangsseite des Bildsensors in Richtung senkrecht zu dieser. Bei dem aus EP 2 018 043 A1 (Fig. 4a, 4b) bekannten Bildaufnehmermodul sind die Kontaktfinger zusätzlich nochmals an ihrem äußersten Ende rechtwinkelig umgebogen, was zu einem Abbrechen der Kontaktfinger führen kann. Der weitere Vorteil der vorstehend genannten Maßnahme besteht darin, dass vor dem Anbringen des Bildsensors an der Anordnung aus erster und zweiter Platine an den zweiten Kontaktstellen ein Funktionstest aller leitfähigen Verbindungen durchgeführt werden kann, ohne dass dazu zuvor der Bildsensor mit der Anordnung aus erster Platine und zweiter Platine elektrisch kontaktiert werden muss.

Im Zusammenhang mit der vorstehend genannten Maßnahme sind die zweiten Kontaktstellen als sich in Richtung von der dem Bildsensor zugewandten Oberfläche zur gegenüberliegenden Oberfläche der ersten Platine erstreckende leitfähige Vertiefungen an der zumindest einen Schmalseite ausgebildet.

Hierbei ist von Vorteil, dass die Kontaktfinger mittels in die leitfähigen Vertiefungen eingebrachten leitfähigen Klebstoffs oder Lots auf einfache Weise mit der ersten Platine kontaktiert werden können.

Vorzugsweise sind die leitfähigen Vertiefungen als im Wesentlichen halb aufgeschnittene Zylinder ausgebildet.

Hierbei ist von Vorteil, dass die zweiten Kontaktstellen an der ersten Platine ebenfalls auf einfache Weise hergestellt werden können. So können beispielsweise in die erste Platine, die zunächst mit einem Übermaß gefertigt wird, in einer oder zwei Reihen an gegenüberliegenden Randbereichen der ersten Platine durchgehende Bohrungen eingebracht werden, und durch Abschneiden der Ränder der Platine etwa mittig durch die Bohrungen hindurch entstehen dann die Halbzylinder, die nur noch zu metallisieren sind, wenn die Metallisierung nicht bereits in die Bohrungen eingebracht wurde.

In einer weiteren bevorzugten Ausgestaltung ist die erste Platine mit den Adern an den ersten Kontaktstellen mittels leitfähigen Fügepunkten kontaktiert und verbunden.

Diese Maßnahme hat den Vorteil, dass die erste Platine mit der zweiten Platine über die ersten Kontaktstellen nicht nur elektrisch leitend kontaktiert ist, sondern es wird auch eine mechanische Verbindung über die Fügepunkte, beispielsweise Klebstoff- oder Lotpunkte, zwischen den beiden Platinen hergestellt, ohne dass weitere Maßnahmen für eine mechanische Verbindung der beiden Platinen aneinander getroffen werden müssen. Außerdem kann das Aufbringen der Fügepunkte entweder auf die freiliegenden Enden der Adern oder umgekehrt auf die ersten Kontaktstellen der ersten Platine automatisiert mittels eines Roboters erfolgen. Nach dem Aufbringen der Fügepunkte muss die erste Platine dann lediglich noch auf die zweite Platine aufgesetzt werden, was ebenfalls mittels eines Roboters auf automatisierte Weise durchgeführt werden kann.

In einer alterantiven Ausgestaltung ist zwischen der ersten Platine und der zweiten Platine im Bereich der ersten Kontaktstellen ein flächiges Element angeordnet, das aus einem isolierenden komprimierbaren Material gefertigt ist, das mit leitenden Partikeln durchsetzt ist, wobei das flächige Element an den ersten Kontaktstellen lokal komprimiert ist, um die erste Platine mit den Adern elektrisch leitend zu kontaktieren.

Ein solches flächiges Element, das hier verwendet werden kann, ist ein sog. Leitgummi. Das Leitgummi weist als Trägermaterial Gummi auf, das von leitenden Partikeln durchsetzt ist. Durch Komprimieren des Gummis kommen die leitenden Partikel, beispielsweise Kohle- oder Rußpartikel, miteinander in Berührung und erzeugen somit jeweils einen Leiterpfad zwischen der jeweiligen einzelnen Ader und der zugehörigen ersten Kontaktstelle der ersten Platine. In den Richtungen parallel zur Ebene des flächigen Elements bleibt dieses jedoch isolierend, wodurch nur die zueinander zugehörige Ader und erste Kontaktstelle elektrisch miteinander leitend kontaktiert sind und keine Kurzschlusspfade entstehen.

In einer weiteren bevorzugten Ausgestaltung sind an der der zweiten Platine zugewandten Oberfläche der ersten Platine Abstandhalter vorhanden, die einen schmalen Spalt zwischen den einander zugewandten Oberflächen der ersten Platine und der zweiten Platine definieren.

Diese Maßnahme ist insbesondere im Zusammenhang mit der zuvor genannten Maßnahme von Vorteil, wenn nämlich die beiden Platinen mittels Fügepunkten miteinander kontaktiert und verbunden werden. Durch das Vorsehen von Abstandhaltern zwischen den beiden Platinen wird stets ein definierter und von Bildaufnehmermodul zu Bildaufnehmermodul reproduzierbarer Fügespalt erzeugt. Es wird insbesondere vermieden, dass beim Aufsetzen der ersten Platine auf die zweite Platine die Fügepunkte soweit zusammengedrückt werden, dass sie untereinander verschmieren. Die Abstandhalter können an den ersten Kontaktstellen ausgebildet sein. Die Abstandhalter können beispielsweise in Form von sogenannten Ballbonds an der ersten Platine ausgebildet sein. Solche "Ballbonds" können ebenfalls in automatisierter Weise mittels eines Roboters an der ersten Platine aufgebracht werden.

In einer weiteren bevorzugten Ausgestaltung sind die Adern in den Bohrungen der zweiten Platine mittels Klebstoffs fixiert.

Diese Maßnahme hat zum einen den Vorteil, dass für das Kabel eine Zugentlastung geschaffen wird, weil nämlich die einzelnen Adern in den Bohrungen durch den Klebstoff mechanisch fixiert sind. Zum anderen hat diese Maßnahme bei der Assemblierung den Vorteil, dass die Adern nach dem Einführen in die Bohrungen zunächst mittels des Klebstoffs fixiert werden können, und das oben erwähnte Schneiden entlang der Oberfläche der zweiten Platine senkrecht zu den Adern danach erfolgt, so dass beim Schneiden die Adern in den Bohrungen lagefixiert sind und es somit nicht vorkommen kann, dass einzelne Adern beim Schneiden aus den Bohrungen heraus- oder in die Bohrungen zurückgezogen werden können.

In einer weiteren bevorzugten Ausgestaltung schließen die Enden der Adern mit ihren Aderseelen und ihren Adermänteln mit den Rändern der Bohrungen an der der ersten Platine zugewandten Oberfläche der zweiten Platine etwa bündig ab.

Hierbei ist von Vorteil, dass für alle Adern die Lage der Enden der Adern stets wohl definiert ist, wodurch beim Kontaktieren der ersten Platine mit der zweiten Platine stets gewährleistet ist, dass die ersten Kontaktstellen mit den zugehörigen Adern zuverlässig elektrisch leitend verbunden werden.

In einer weiteren bevorzugten Ausgestaltung ist der Bildsensor von der ersten Platine beabstandet, wobei der Raum zwischen dem Bildsensor und der Platine mit einer Vergussmasse ausgefüllt ist.

Die Verwendung einer aushärtenden Vergussmasse bei Bildaufnehmermodulen ist an sich bekannt. Beim erfindungsgemäßen Bildaufnehmermodul hat die Verwendung der Vergussmasse jedoch nicht nur den Vorteil des Schutzes der elektrischen oder elektronischen Bauteile auf der ersten Platine, sondern erleichtert auch das Assemblieren des Bildsensors mit der Anordnung aus der ersten und zweiten Platine. Die Vergussmasse kann nämlich vor dem Anbringen des Bildsensors an der ersten Platine auf die Oberfläche der ersten Platine mit einer vorbestimmten Dicke aufgebracht werden, so dass nach dem Aushärten der Vergussmasse der Bildsensor durch Aufsetzen auf die Vergussmasse dann bereits den vorbestimmten Abstand zur Oberfläche der ersten Platine aufweist und auch die Kontaktfinger dann in der richtigen Länge in Bezug auf die zweiten Kontaktstellen angeordnet sind.

In einer weiteren bevorzugten Ausgestaltung ist das zumindest eine elektronisch elektrische Bauelement auf der dem Bildsensor zugewandten Oberfläche der ersten Platine angeordnet.

Die Anordnung des zumindest einen elektronischen oder elektrischen Bauelements auf der dem Bildsensor zugewandten Oberfläche der ersten Platine hat den Vorteil, dass die erste Platine und die zweite Platine mit einem sehr geringen Spaltabstand zueinander elektrisch kontaktiert und miteinander verbunden werden können.

Die an zweiter Stelle genannte Aufgabe wird erfindungsgemäß durch ein Verfahren zum Herstellen eines Bildaufnehmermoduls gemäß dem Anspruch 9 gelöst.

Auf die erste Platine wird eine Vergussmasse aufgebracht, wobei der Bildsensor auf die Vergussmasse aufgesetzt und die Kontaktfinger des Bildsensors an zweiten Kontaktstellen, die an einer Schmalseite der ersten Platine angeordnet sind, mit der ersten Platine kontaktiert werden.

Wie bereits oben in Bezug auf das erfindungsgemäße Bildaufnehmermodul beschrieben, kann das vorstehend genannte erfindungsgemäße Verfahren insbesondere automatisiert, insbesondere mittels eines Roboters, durchgeführt werden.

Die Adern des Kabels werden vorzugsweise mit ihren Adermänteln in die Bohrung eingeführt, bis die Adern mit ihren Adermänteln aus den Bohrungen vorstehen, wobei die Adern in den Bohrungen mittels Klebstoff fixiert werden, und wobei entlang der Oberfläche der zweiten Platine senkrecht zu und durch die Adern geschnitten wird, so dass die Enden der Adern mit Rändern der Bohrungen an der Oberfläche der zweiten Platine etwa bündig abschließen.

Wie bereits oben erwähnt, entfällt durch diese Maßnahme das Abisolieren der Adern, um die Aderseelen für die spätere Kontaktierung mit den ersten Kontaktstellen der ersten Platine freizulegen. Durch das Schneiden senkrecht zu und durch die Adern werden nämlich die Aderseelen stirnseitig stumpf freigelegt, d.h. sichtbar und kontaktierbar.

Vorzugsweise werden auf die Enden der Adern oder auf die ersten Kontaktstellen leitfähige Fügepunkte aufgebracht, wobei die ersten Kontaktstellen der ersten Platine auf die Fügepunkte aufgesetzt werden, um die erste Platine mit den Adern zu kontaktieren.

Bei dem vorstehend genannten Schritt findet nicht nur eine elektrisch leitfähige Kontaktierung der ersten Platine mit der zweiten Platine statt, sondern auch eine mechanische Verbindung bzw. Fixierung der beiden Platinen aneinander. Der vorstehend genannte Schritt kann insbesondere automatisiert mittels eines Roboters durchgeführt werden.

Alternativ zu der vorstehend genannten Vorgehensweise wird auf der zweiten Platine im Bereich der Adern ein flächiges Element angeordnet, das aus einem isolierenden komprimierbaren Material gefertigt ist, das partiell mit leitenden Partikeln durchsetzt ist, wobei die erste Platine auf das flächige Element gedrückt und an der zweiten Platine festgelegt wird.

Wie bereits oben erwähnt, kann das flächige Element ein Leitgummi sein, das beispielsweise mit Kohle- oder Rußpartikeln durchsetzt ist. Das Festlegen der ersten Platine an der zweiten Platine kann dabei beispielsweise mittels eines Klebstoffs erfolgen, der beispielsweise nur an den Ecken der ersten und zweiten Platine aufgebracht wird, wobei an diesen Klebepunkten dann das flächige Element nicht vorhanden oder zumindest ausgespart ist, damit die erste Platine und die zweite Platine unter Druckausübung auf das flächige Element aneinander festgelegt werden können.

Weiter vorzugsweise wird vor dem Aufbringen der Vergussmasse und vor dem Aufsetzen des Bildsensors an den zweiten Kontaktstellen die Funktion der leitfähigen Verbindungen der ersten und zweiten Platine getestet.

Durch das erfindungsgemäße Verfahren wird nicht nur die Möglichkeit geschaffen, das Assemblieren des Bildaufnehmermoduls automatisiert mittels eines Roboters durchzuführen, sondern das erfindungsgemäße Verfahren und auch das erfindungsgemäße Bildaufnehmermodul haben den Vorteil, dass der Funktionstest aller leitfähigen Verbindungen der ersten und zweiten Platine vor dem Anbringen des Bildsensors durchgeführt werden kann, so dass fehlerhafte Platinenanordnungen ausgesondert werden können und es somit nicht zu einer Verschwendung teuerer Bildsensoren kommt, weil diese für den Funktionstest nicht an den Platinen angebracht sein müssen.

Weitere Vorteile und Merkmale ergeben sich aus der nachfolgenden Beschreibung und der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel eines erfindungsgemäßen Bildaufnehmermoduls und eines erfindungsgemäßen Verfahrens zu seiner Herstellung werden nachfolgend mit Bezug auf die beigefügte Zeichnung beschrieben. Es zeigen:
- Fig. 1: ein Bildaufnehmermodul in einer perspektivischen Gesamtansicht, wobei das Bildaufnehmermodul stark vergrößert dargestellt ist;
- Fig. 2: eine auseinandergezogene perspektivische Darstellung des Bildaufnehmermoduls in Fig. 1;
- Fig. 3: eine perspektivische Ansicht auf eine Terminalplatine des Bildaufnehmermoduls in Fig. 1 mit angeschlossenem mehradrigem Kabel; und
- Fig. 4: eine perspektivische Ansicht von unten auf die Terminalplatine in Fig. 3 und auf eine Komponentenplatine des Bildaufnehmermoduls in Fig. 1, wobei die Terminalplatine und die Komponentenplatine auseinandergezogen dargestellt sind.

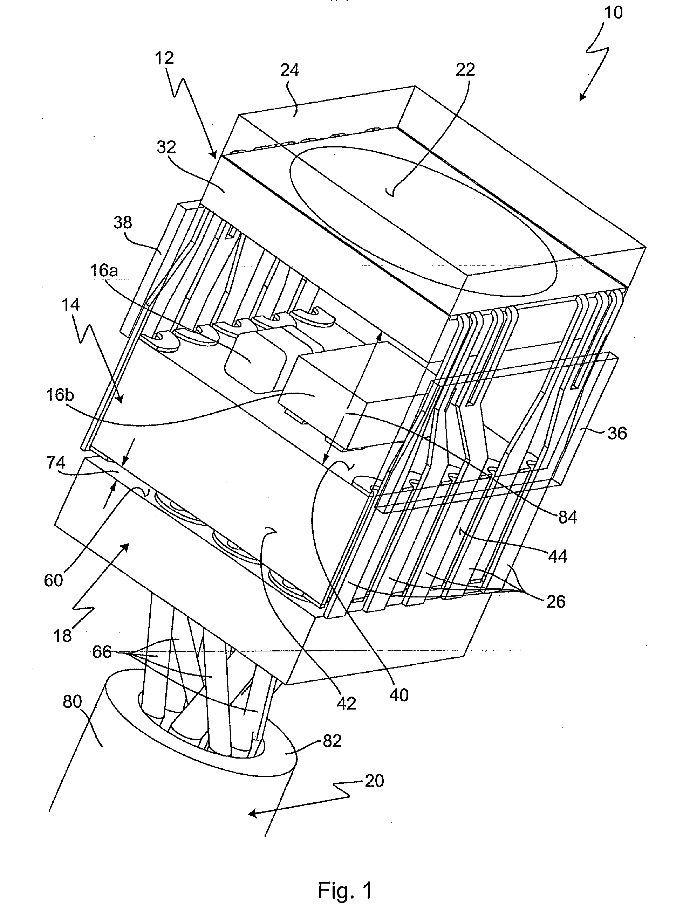

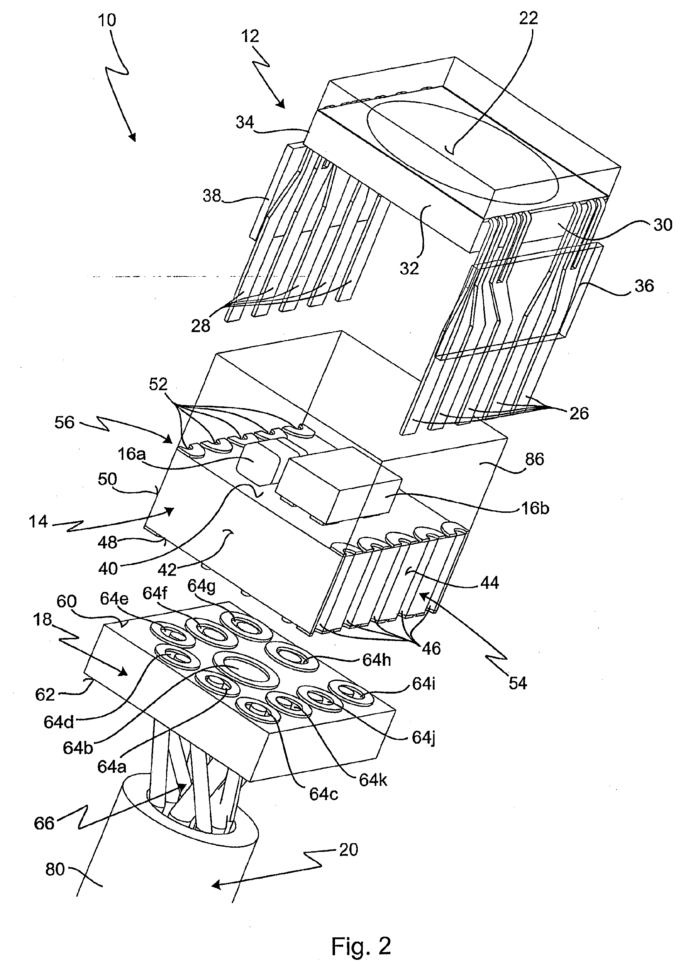

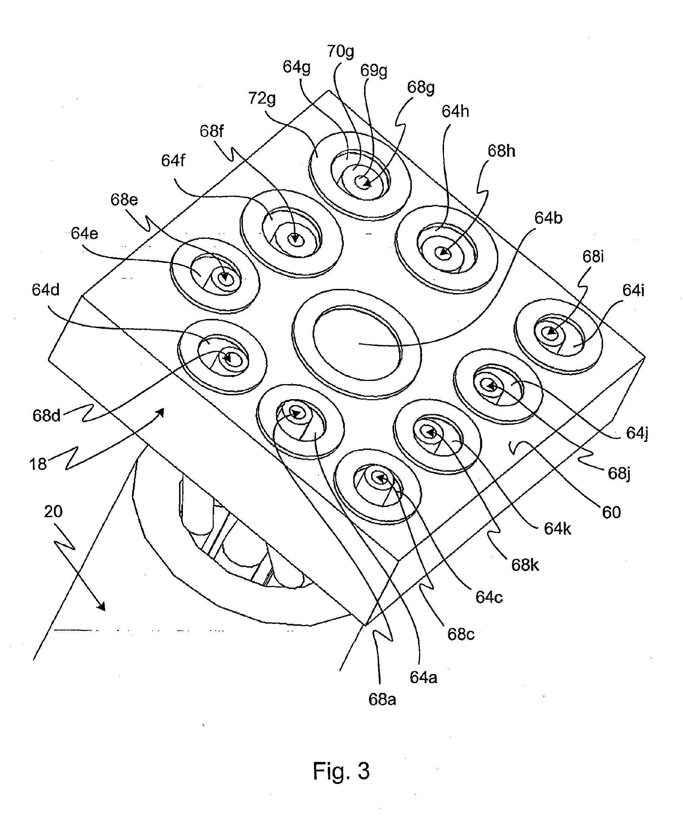

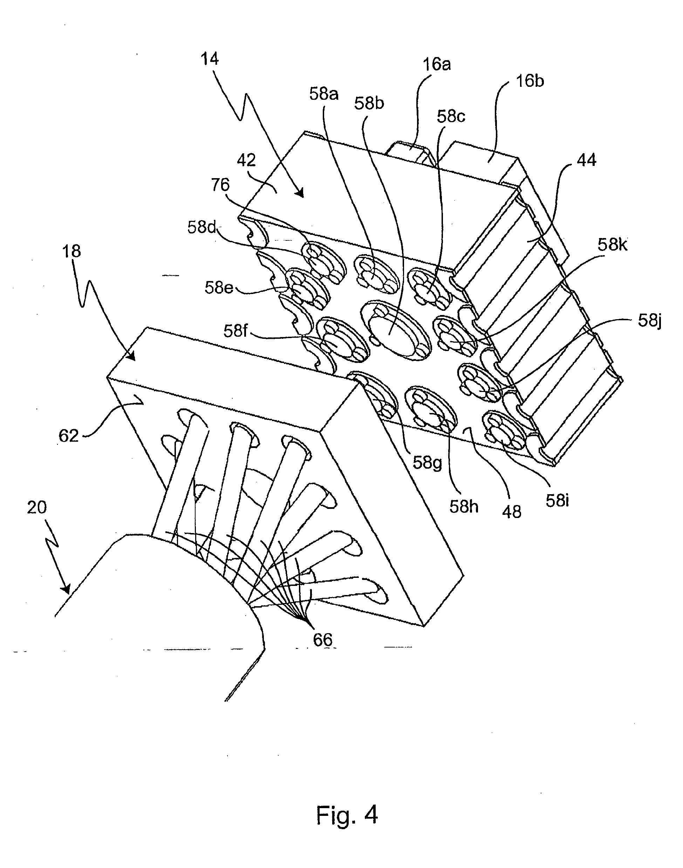

In Fig. 1 ist ein mit dem allgemeinen Bezugszeichen 10 versehenes Bildaufnehmermodul im assemblierten Zustand gezeigt. Fig. 2 zeigt das Bildaufnehmermodul 10 in einer Darstellung, in der die einzelnen Hauptbaugruppen des Bildaufnehmermoduls 10 teilweise auseinandergezogen dargestellt sind. Fig. 3 und 4 zeigen weitere Einzelheiten des Bildaufnehmermoduls 10.

Das Bildaufnehmermodul 10 kann bspw. in die distale Spitze eines Endoskops oder in eine medizinische Miniaturkamera, ein Exoskop, etc. eingebaut werden.

Das Bildaufnehmermodul 10 weist gemäß Fig. 1 und 2 als Hauptbaugruppen einen elektronischen Bildsensor 12, eine erste Platine 14, die auch als Komponentenplatine bezeichnet wird und mit zumindest einem, im gezeigten Ausführungsbeispiel zwei elektronischen oder elektrischen Bauelementen 16a, 16b bestückt ist, eine zweite Platine 18, die auch als Terminalplatine bezeichnet wird, und ein mehradriges Kabel 20 auf.

Das Bildaufnehmermodul 10 ist in Fig. 1 und 2 stark vergrößert dargestellt. Es versteht sich, dass das Bildaufnehmermodul 10 ein opto-elektronisches Bauteil in miniaturisierter Form ist.

Nachfolgend werden die einzelnen Hauptbaugruppen des Bildaufnehmermoduls 10 beschrieben.

Der elektronische Bildsensor 12 ist in CCD- oder CMOS-Technologie in TAB-Konfiguration ausgebildet.

Der Bildsensor 12 weist eine Lichtempfangsfläche 22 auf, auf der ein Deckglas 24 angebracht ist. Durch das transparente Deckglas 24 fällt im Gebrauch des Bildaufnehmermoduls 10 Licht auf die Lichtempfangsfläche 22 ein, wobei die empfangenen Lichtsignale im Bildsensor 12 in elektrische Signale umgewandelt werden. Im Einbauzustand des Bildaufnehmermoduls 10, beispielsweise in einem Endoskop in dessen distaler Spitze, ist dem Bildaufnehmermodul 10 ein Objektiv vorgeschaltet, um ein zu beobachtendes Objekt auf der Lichtempfangsfläche 22 des Bildsensors 12 abzubilden.

Der Bildsensor 12 weist eine Mehrzahl an Kontaktfingern 26, 28 auf. Die Kontaktfinger 26 sind in einer Reihe an einer Seite 30 eines Grundkörpers 32 des Bildsensors 12 angeordnet, und die Kontaktfinger 28 sind in einer Reihe an einer Seite 34, die der Seite 30 gegenüberliegt, des Grundkörpers 32 angeordnet.

Die Kontaktfinger 26 und 28 erstrecken sich senkrecht zur Lichtempfangsfläche 22 des Bildsensors 12 zur ersten Platine 14 hin.

Die Kontaktfinger 26, die ihrerseits elektrisch leitfähig sind, sind untereinander durch ein elektrisch nicht leitfähiges plattenförmiges Element 36 mechanisch stabilisiert. Ebenso sind die Kontaktfinger 28, die ihrerseits elektrisch leitfähig sind, durch ein elektrisch nicht-leitfähiges plattenförmiges Element 38 relativ zueinander mechanisch stabilisiert.

Die erste Platine 14, die mit den elektronischen oder elektrischen Bauelementen 16a, 16b bestückt ist, weist insgesamt die Form eines flachen Quaders auf. Die erste Platine 14 weist eine dem Bildsensor 12 zugewandte Oberfläche 40 auf, auf der die elektrischen oder elektronischen Bauelemente 16a, 16b angeordnet und elektrisch kontaktiert sind. Die Oberfläche 40 und damit auch die erste Platine 14 insgesamt sind im assemblierten Zustand des Bildaufnehmermoduls 10 parallel zur Lichtempfangsfläche 22 des Bildsensors 12 orientiert.

Die erste Platine 14 weist weiterhin vier Schmalseiten auf, von denen in Fig. 1 und 2 zwei zu sehen sind, die mit dem Bezugszeichen 42 und 44 versehen sind.

Die Oberfläche 40 der Platine 14 weist beispielsweise eine Kantenabmessung von etwa 2 mm auf, während die Höhe der Schmalseiten 42 bzw. 44 in Richtung senkrecht zur Oberfläche 40 etwa 1 mm oder darunter beträgt.

An der Schmalseite 44 der ersten Platine 14 sind entsprechend der Anzahl an Kontaktfingern 26 eine gleiche Anzahl an leitfähigen Vertiefungen 46 vorhanden. Die Vertiefungen sind als im Wesentlichen halb aufgeschnittene Zylinder ausgebildet, wie insbesondere aus Fig. 2 hervorgeht. Die Vertiefungen 46 erstrecken sich von der Oberfläche 40 der ersten Platine 14 zu einer gegenüberliegenden Oberfläche 48 der ersten Platine 14 (siehe auch Fig. 4). Die Leitfähigkeit der Vertiefungen 46 wird durch eine entsprechende Metallisierung der Vertiefungen 46, beispielsweise mit Gold, erzielt. Die halbzylindrischen Vertiefungen 46 können bei der Herstellung der ersten Platine 14 dadurch erzeugt werden, dass die erste Platine 14 zunächst mit einem Übermaß in Richtung der Schmalseite 42 hergestellt wird, in den Randbereich der Schmalseite 44 dann durchgehende Bohrungen eingebracht werden, und durch Schneiden der Platine 14 auf Maß, wobei der Schnitt etwa mittig durch die vorstehend beschriebenen Bohrungen erfolgt, werden dann die halbzylindrischen Vertiefungen 46 erzeugt. Die Metallisierung der Vertiefungen 46 kann dabei bereits nach dem Einbringen der Bohrungen erfolgen oder erst nach dem Aufschneiden der Bohrungen.

Auf einer der Schmalseite 44 gegenüberliegenden Schmalseite 50 sind ebenfalls leitfähige Vertiefungen 52 in Form von im Wesentlichen halb aufgeschnittenen Zylindern ausgebildet, die sich von der Oberfläche 40 zur gegenüberliegenden Oberfläche 48 durchgehend erstrecken.

Die Vertiefungen 46 dienen als Kontaktstellen zum Kontaktieren der Kontaktfinger 26 mit der ersten Platine 14. Die Vertiefungen 52 dienen als Kontaktstellen 56 zum Kontaktieren der Kontaktfinger 28 des Bildsensors 12 mit der ersten Platine 14.

Im in Fig. 1 gezeigten assemblierten Zustand des Bildaufnehmermoduls 10 sind die Kontaktfinger 26 mittels eines leitfähigen Klebers an bzw. in den Vertiefungen 46 ein- bzw. angeklebt, und die Kontaktfinger 28 sind entsprechend in bzw. an die Vertiefungen 52 an- bzw. eingeklebt, um die Kontaktfinger 28 mit der ersten Platine leitfähig zu kontaktieren.

An der dem Bildsensor 12 abgewandten Oberfläche 48 weist die erste Platine 14 eine Mehrzahl an punktförmigen Kontaktstellen auf, die mit den Bezugszeichen 58a bis 58k versehen sind. Die Kontaktstellen 58a bis 58k weisen keine oder allenfalls eine minimale Erhebung von der Oberfläche 48, bspw. im µ-Bereich, auf.

Die erste Platine 14 ist in einem Mehrschichtaufbau gefertigt, wobei sich durch das Innere der Platine 14 entsprechend elektrische Leitungen erstrecken, so dass die punktförmigen Kontaktstellen 58a bis 58k teilweise mit den leitfähigen Vertiefungen 46 und 52 und teilweise mit den elektrischen oder elektronischen Bauelementen 16a und 16b elektrisch leitend verbunden sind. Die elektrischen Bauelemente 16a und 16b sind Teil der Steuerelektronik des Bildsensors 12.

Die zweite Platine 18, die auch als Terminalplatine bezeichnet wird, weist ebenfalls die Form eines flachen Quaders auf, wobei die Höhe der zweiten Platine 18 in Richtung senkrecht zur Lichtempfangsfläche 22 des Bildsensors 12 noch geringer ist als die Höhe der ersten Platine 14.

Die zweite Platine 18 dient lediglich der Terminierung des Kabels 20.

Die zweite Platine 18 weist eine der ersten Platine 14 zugewandte Oberfläche 60 und eine dieser gegenüberliegende Oberfläche 62 (siehe auch Fig. 4) auf. Die Oberfläche 60 bzw. die zweite Platine 18 als solche verläuft im assemblierten Zustand des Bildaufnehmermoduls 10 parallel zur Lichtempfangsfläche 22 des Bildsensors 12 und parallel zur Platine 14.

Die Oberfläche 60 der zweiten Platine 18 ist wie die der Oberfläche 60 zugewandte Oberfläche 48 der ersten Platine 14 im Wesentlichen eben.

Die zweite Platine 18 weist eine Mehrzahl an Bohrungen 64a bis 64k auf, die jeweils von der Oberfläche 62 zur gegenüberliegenden Oberfläche 60 der zweiten Platine 18 durchgehend sind. Die Bohrungen 64a bis 64k sind über die zweite Platine 18 zwischen Rändern derselben verteilt.

Die Flächenverteilung der Bohrungen 64a bis 64k in der zweiten Platine 18 entspricht dabei der Flächenverteilung der Kontaktstellen 58a bis 58k an der Oberfläche 48 der ersten Platine 14. Mit anderen Worten ist die Belegung der Oberfläche 48 mit den Kontaktstellen 58a bis 58k spiegelbildlich zur Belegung der Platine 18 mit den Bohrungen 64a bis 64k.

Jeder Bohrung 64a bis 64k ist somit unmittelbar gegenüberliegend eine der Kontaktstellen 58a bis 58k zugeordnet, d.h. der Bohrung 64a ist die Kontaktstelle 58a, der Bohrung 64b die Kontaktstelle 58b, der Bohrung 64c die Kontaktstelle 58c, usw. unmittelbar gegenüberliegend zugeordnet, wenn das Bildaufnehmermodul 10 assembliert ist.

Das mehradrige Kabel 20 weist eine Mehrzahl an Adern 66 auf.

Die Adern 66 sind in die Bohrungen 64a bis 64k der zweiten Platine 18 eingeführt, wobei Enden 68a bis 68k der Adern 66 (die Ader bzw. Adern 68b sind in der Zeichnung nicht dargestellt, jedoch genauso in der Bohrung 64b vorhanden) an der Oberfläche 60 der zweiten Platine 18 enden, wobei die Enden 68a bis 68k im Wesentlichen bündig mit der Oberfläche 60 der zweiten Platine 18 stumpf abgeschnitten sind.

Die Enden 68a bis 68k der Adern 66 weisen Adermäntel und stirnseitig freiliegende Aderseelen auf, wie für eine Aderseele 69g und einen Adermantel 70g des Endes 68g der entsprechenden Ader in Fig. 3 dargestellt ist.

Die Ränder der Bohrungen 64a bis 64k in der zweiten Platine 18 sind mit Metallisierungen, beispielsweise Goldringen, belegt, wie für einen Goldring 72g der Bohrung 64g in Fig. 3 dargestellt ist. Anstelle des Materials Gold kann auch ein anderes Metallisierungsmaterial verwendet werden, oder es können auch keine derartigen Metallisierungen vorgesehen sein.

Die erste Platine 14 ist über die punktförmigen Kontaktstellen 58a bis 58k mit den Enden 68a bis 68k der Adern 66 des Kabels 20 elektrisch leitend, nach Signalen getrennt, kontaktiert.

Die Kontaktierung der ersten Platine 14 mit den Adern 66 des Kabels 20 ist mittels leitfähigen Fügepunkten an den Kontaktstellen 58a bis 58k bewerkstelligt. Solche Fügepunkte sind vorzugsweise als Klebstoff- oder Lotpunkte ausgeführt. Auf diese Weise ist die erste Platine 14 nicht nur mit den Adern 66 des Kabels 20 elektrisch leitend kontaktiert, sondern die erste Platine 14 ist mit der zweiten Platine 18 über die Fügepunkte auch mechanisch verbunden.

Alternativ zur Kontaktierung der ersten Platine 14 mit den Adern 66 des Kabels 20 mittels leitfähiger Fügepunkte kann es auch vorgesehen sein, zwischen der ersten Platine 14 und der zweiten Platine 18 ein Leitgummi vorzusehen, das im Bereich jeder einzelnen Kontaktstelle 58a-58k lokal komprimiert ist, und durch die lokale Kompression zwischen der jeweiligen Kontaktstelle 58a-58k und der zugehörigen Ader 66 elektrisch leitfähig ist. In Richtung parallel zu den Ebenen der Platine 14 und der Platine 18 bleibt das Leitgummi dagegen isolierend. Bei der Verwendung eines Leitgummis ist die erste Platine 14 mit der zweiten Platine 18, beispielsweise durch Zusammenkleben an beispielsweise den vier Ecken fest verbunden, an denen das Leitgummi nicht vorhanden oder für diese Zwecke ausgespart ist.

Die zuvor erwähnten Goldringe 72 dienen der Vergrößerung der Kontaktstellen, um eine sichere Kontaktierung der Kontaktstellen 58a bis 58k mit den Adern 66 des Kabels 20 zu gewährleisten.

Wie aus Fig. 1 hervorgeht, sind die erste Platine 14 und die zweite Platine 18 im assemblierten Zustand des Bildaufnehmermoduls 10 nur um einen sehr dünnen Spalt 74 voneinander beabstandet, der im µ-Bereich liegen kann. Um den Spalt 74 in definierter und reproduzierbarer Weise beim Assemblieren des Bildaufnehmermoduls 10 einzustellen, sind an der Oberfläche 48 der Platine 14, hier an manchen oder allen der Kontaktstellen 58a bis 58k Abstandhalter 76 (siehe Fig. 4) angeordnet. In dem gezeigten Ausführungsbeispiel sind dabei jeder der Kontaktstellen 58a bis 58k drei Abstandhalter 76 in Form von kleinen sog. Ballbonds zugeordnet. Die Abstandhalter 76 verhindern beim Assemblieren des Bildaufnehmermoduls 10, wenn die erste Platine 14 mit den ersten Kontaktstellen 58a bis 58k auf die zweite Platine 18 aufgesetzt wird, dass die vorstehend genannten Klebstoff- oder Lotpunkte übermäßig komprimiert und damit entlang der Oberfläche 48 der ersten Platine 14 bzw. der Oberfläche 60 der zweiten Platine 18 verschmiert werden, so dass Kurzschlüsse zwischen einzelnen Kontaktstellen 58a bis 58k vermieden werden, bzw. eine nach Signalen getrennte Kontaktierung der ersten Platine 14 mit der zweiten Platine 18 gewährleistet wird.

Die einzelnen Adern 66 des mehradrigen Kabels 20 sind des Weiteren in den Bohrungen 64a bis 64k mittels eines Klebstoffs fixiert, wodurch auch eine Zugentlastung des Kabels 20 geschaffen wird.

Das Kabel 20 weist einen Kabelmantel 80 auf, wobei die zweite Platine 18 so weit wie möglich an die durch den Rand 82 des Kabelmantels 80 gebildete Kabelwurzel herangeführt ist.

Das erfindungsgemäße Bildaufnehmermodul 10 weist in Richtung senkrecht zur Lichtempfangsfläche 22 des Bildsensors eine sehr geringe axiale Baulänge auf, wie es für den Einbau des Bildaufnehmermoduls 10 in die distale Spitze eines flexiblen Endoskops erwünscht ist.

Wie aus Fig. 1 hervorgeht, sind die elektrischen oder elektronischen Bauelemente 16a, 16b auf der dem Bildsensor 12 zugewandten Oberfläche 40 der ersten Platine 14 angeordnet. Der Bildsensor 12 ist von der Oberfläche 40 um einen Abstand 84 beabstandet, wobei der Raum zwischen dem Bildsensor 12, genauer gesagt dessen der Oberfläche 40 zugewandten Oberfläche, und der Oberfläche 40 mit einer Vergussmasse 86 ausgefüllt ist (siehe Fig. 2), wobei die Vergussmasse 86 hier zur Vereinfachung der Darstellung transparent dargestellt ist, es sich jedoch versteht, dass die Vergussmasse auch nicht transparent sein kann.

Nachfolgend wird ein beispielhaftes Verfahren zur Herstellung des Bildaufnehmermoduls 10 beschrieben.

In einem ersten Schritt werden die zweite Platine 18 mit den darin befindlichen Bohrungen 64a bis 64k und das mehradrige Kabel 20 bereitgestellt.

Im nächsten Schritt wird der Kabelmantel 80 des Kabels 20 über eine gewisse Länge abgenommen, wodurch die einzelnen Adern 66 freigelegt werden. Die sich auf der Innenseite des Kabelmantels 80 befindliche Gesamtabschirmung wird nach hinten gezogen. Aus den Abschirmungen der einzelnen Adern 66 wird eine weitere Ader oder Litze geformt. Alle Adern 66 (einschließlich der durch die Abschirmungen geformten Ader) werden dann entsprechend dem Verschaltungsplan in die Bohrungen 64a bis 64k eingeführt bzw. eingefädelt, bis die Enden 68a bis 68k von der Oberfläche 60 der zweiten Platine 18 vorstehen. Es versteht sich, dass es von dem Verschaltungsplan abhängig ist, ob durch jeweils eine der Bohrungen 64a bis 64k nur eine Ader der Adern 66 eingeführt wird oder ggf. auch zwei oder mehrere der Adern 66 eingeführt werden.

Die zweite Platine 18 wird so weit wie möglich an die Kabelwurzel (Rand 82 des Kabelmantels 80) herangeschoben.

Die Enden 68a bis 68k stehen nun von der Oberfläche 60 der zweiten Platine 18 vor.

Im nächsten Schritt werden die Adern 66 in den Bohrungen 64a bis 64k mittels eines Klebstoffs mechanisch fixiert.

Die durch die Bohrungen 64a bis 64k von der Oberfläche 60 der zweiten Platine 18 vorstehenden Enden 68a bis 68k der Adern 66 weisen neben den Aderseelen 69, die elektrisch leitfähig sind, auch die Adermäntel 70 auf. Es versteht sich, dass die vorstehend genannte weitere Ader, die aus den Abschirmungen der einzelnen Adern 66 geformt wurde, keinen Adermantel aufweist.

Im nächsten Schritt wird nun mittels einer geeigneten Vorrichtung parallel zur Oberfläche 60 und senkrecht zu und durch die überstehenden Enden 68a bis 68k der Adern 66 geschnitten, so dass die Enden 68a bis 68k mit ihren Aderseelen 69 und ihren Adermänteln 70 im Wesentlichen bündig mit der Oberfläche 60 der zweiten Platine 18 abschließen. Das Ablängen der einzelnen Adern 66 auf die richtige Länge kann somit automatisiert mittels eines Roboters durchgeführt werden, und zwar ohne dass zuvor wie bei den bekannten Verfahren die einzelnen Adern einzeln abgelängt und abisoliert werden müssen. Eine Abisolierung der Adern erübrigt sich vollständig.

Die Enden 68a bis 68k der Adern 66 sind nun stumpf abgelängt und die elektrisch leitenden Aderseelen 69 sind nun stirnseitig freigelegt und kontaktierbar.

Im nächsten Schritt werden auf die Enden 68a bis 68k an der Oberfläche 60 der zweiten Platine Klebepunkte aus einem leitfähigen Klebstoff aufgebracht. Auch dieser Vorgang kann automatisiert mittels eines Roboters durchgeführt werden.

Im nächsten Schritt wird die bereits zuvor mit den elektrischen oder elektronischen Bauelementen 16a, 16b bestückte erste Platine 14 auf die zweite Platine 18 aufgesetzt, so dass die erste Platine 14 über die Kontaktstellen 58a bis 58k mit den Enden 68a bis 68k der Adern 66 elektrisch leitend kontaktiert wird. Auch das Aufsetzen der Platine 14 auf die Platine 18 kann automatisiert mittels eines Roboters durchgeführt werden.

Nach dem Aushärten der Klebepunkte sind die erste Platine 14, die zweite Platine 18 und das Kabel 20 nach Signalen getrennt miteinander verbunden. Nach dem Aushärten der Klebepunkte ist die erste Platine 14 mit der zweiten Platine 18 auch mechanisch fixiert verbunden.

Nun kann durch Messung an den leitfähigen Vertiefungen 46 und 52 der ersten Platine 14 die Funktion aller leitfähigen Verbindungen der assemblierten Baugruppe aus erster Platine 14, zweiter Platine 18 und Kabel 20 getestet werden, und es können etwaige Schlechtteile ausgesondert werden, und zwar bevor der Bildsensor 12 mit der ersten Platine 14 verbunden wird.

Im nächsten Schritt wird der Bildsensor 12 aus seiner Lieferverpackung mittels Laserlicht herausgetrennt. In diesem Zustand verlaufen die Kontaktfinger 26 und 28 noch parallel zur Lichtempfangsfläche 22 des Bildsensors 12. Die Kontaktfinger 26 und 28 werden dann etwa senkrecht zur Lichtempfangsfläche 22 des Bildsensors 12 umgebogen.

Zuvor wurde auf die Oberfläche 40 der ersten Platine 14 die Vergussmasse 86 in vorbestimmter Dicke (bezogen auf die Richtung senkrecht zur Lichtempfangsfläche 22) aufgebracht, und nach dem Aushärten der Vergussmasse 86 wird der Bildsensor 12 auf die Vergussmasse 86 aufgesetzt. Durch das Vorhandensein der Vergussmasse 86 nimmt der Bildsensor 12 beim Auflegen des Grundkörpers 32 auf die obere Oberfläche der Vergussmasse 86 einen reproduzierbaren, definierten Abstand zur Oberfläche 40 der ersten Platine 14 ein, so dass die Kontaktfinger 26, 28 in der richtigen Länge etwa auf Höhe der Oberfläche 48 der Platine 14 enden.

Im letzten Schritt werden dann die Kontaktfinger 26 und 28 mit den leitfähigen Vertiefungen 46 bzw. 52 der ersten Platine 14 mittels eines leitfähigen Klebstoffs verklebt. Der Bildsensor 12 ist nun mit der ersten Platine 14 elektrisch leitend kontaktiert.

Das Bildaufnehmermodul 10 ist nun fertiggestellt.

Es ergibt sich aus dem Vorstehenden, dass alle zuvor genannten Verfahrensschritte der Assemblierung des Bildaufnehmermoduls 10 automatisiert und vorzugsweise mittels eines Roboters durchgeführt werden können.

## Patentansprüche

1. Bildaufnehmermodul, insbesondere für ein Endoskop, mit:
einem elektronischen Bildsensor (12),
einer ersten Platine (14), die zumindest ein elektronisches oder elektrisches Bauelement (16a, 16b) aufweist, wobei die erste Platine (14) parallel zu einer Lichtempfangsfläche (22) des Bildsensors (12) angeordnet ist,
einer zweiten Platine (18), die auf einer dem Bildsensor (12) abgewandten Seite der ersten Platine (14) parallel zur ersten Platine (14) angeordnet ist, wobei die zweite Platine (18) eine Mehrzahl an Bohrungen (64a-64k) aufweist, die von einer der ersten Platine (14) abgewandten zu einer der ersten Platine (14) zugewandten Oberfläche (60, 62) der zweiten Platine (18) durchgehend und
flächig über die zweite Platine (18) zwischen Rändern der zweiten Platine (18) verteilt sind,
einem Kabel (20), das eine Mehrzahl an Adern (66) aufweist, mit der zweiten Platine (18) verbunden ist und von der der ersten Platine (14) abgewandten Oberfläche (62) der zweiten Platine (18) wegführt, wobei die Adern (66) in die Bohrungen (64a-64k) der zweiten Platine (18) eingeführt sind, und Enden (68a-68k) der Adern sich etwa an der der ersten Platine (14) zugewandten Oberfläche (60) der zweiten Platine (18) befinden,
wobei die erste Platine (14) auf einer der zweiten Platine (18) zugewandten Oberfläche (48) eine Mehrzahl an punktförmigen ersten elektrischen Kontaktstellen (58a-58k) aufweist, deren Flächenverteilung der Flächenverteilung der Enden (68a-68k) der Adern (66) an der Oberfläche (60) der zweiten Platine (18) entspricht, dass die der ersten Platine (14) zugewandte Oberfläche (60) der zweiten Platine (18) insgesamt im Wesentlichen eben ist, und dass die erste Platine (14) über die ersten Kontaktstellen (58a-58k) mit den Enden (68a-68k) der Adern (66) elektrisch leitend kontaktiert ist,
**dadurch gekennzeichnet, dass** der Bildsensor (12) eine Mehrzahl an Kontaktfingern (26, 28) aufweist, die in zumindest einer Reihe angeordnet sind und
sich senkrecht zur Lichtempfangsfläche (22) des Bildsensors (12) zur ersten Platine (14) hin erstrecken, dass die erste Platine (14) an zumindest einer Schmalseite (44, 50) eine Mehrzahl an zweiten Kontaktstellen (54, 56) aufweist, und dass die Kontaktfinger (26, 28) des Bildsensors (12) an den zweiten Kontaktstellen (54, 56) elektrisch leitend kontaktiert sind, und
dass die zweiten Kontaktstellen (54, 56) als sich in Richtung von der dem Bildsensor (12) zugewandten Oberfläche (40) zur gegenüberliegenden Oberfläche (48) der ersten Platine (14) erstreckende leitfähige Vertiefungen (46, 52) an der zumindest einen Schmalseite (44, 50) ausgebildet sind.

2. Bildaufnehmermodul nach Anspruch 1, **dadurch gekennzeichnet, dass** die leitfähigen Vertiefungen (46, 52) als im Wesentlichen halb aufgeschnittene Zylinder ausgebildet sind.

3. Bildaufnehmermodul nach Anspruch 1 oder 2, **dadurch gekennzeichnet, dass** die erste Platine (14) mit den Adern (66) an den ersten Kontaktstellen (58a-58k) mittels leitfähigen Fügepunkten kontaktiert und verbunden ist.

4. Bildaufnehmermodul nach Anspruch 1 oder 2, **dadurch gekennzeichnet, dass** zwischen der ersten Platine (14) und der zweiten Platine (18) im Bereich der ersten Kontaktstellen (58a-58k) ein flächiges Element angeordnet ist, das aus einem isolierenden komprimierbaren Material gefertigt ist, das mit leitenden Partikeln durchsetzt ist, wobei das flächige Element an den ersten Kontaktstellen (58a-58k) lokal komprimiert ist, um die erste Platine (14) mit den Adern (66) elektrisch leitend zu kontaktieren.

5. Bildaufnehmermodul nach einem der Ansprüche 1 bis 4, **dadurch gekennzeichnet, dass** an der der zweiten Platine (18) zugewandten Oberfläche (48) der ersten Platine (14) Abstandhalter (76) vorhanden sind, die einen schmalen Spalt (74) im µm-Bereich zwischen den einander zugewandten Oberflächen (48, 60) der ersten Platine (14) und der zweiten Platine (18) definieren.

6. Bildaufnehmermodul nach einem der Ansprüche 1 bis 5, **dadurch gekennzeichnet, dass** die Adern (66) in den Bohrungen (64a-64k) der zweiten Platine (18) mittels Klebstoffs fixiert sind.

7. Bildaufnehmermodul nach einem der Ansprüche 1 bis 6, **dadurch gekennzeichnet, dass** die Enden (68a-68k) der Adern (66) mit ihren Aderseelen (69) und ihren Adermänteln (70) mit den Rändern der Bohrungen (64a-64k) an der der ersten Platine (14) zugewandten Oberfläche (60) der zweiten Platine (18) etwa bündig abschließen.

8. Bildaufnehmermodul nach einem der Ansprüche 1 bis 7, **dadurch gekennzeichnet, dass** der Bildsensor (12) von der ersten Platine (14) beabstandet ist, und dass der Raum zwischen dem Bildsensor (12) und der Platine (14) mit einer Vergussmasse (86) ausgefüllt ist.

9. Verfahren zum Herstellen eines Bildaufnehmermoduls, insbesondere für ein Endoskop, wobei
ein elektronischer Bildsensor (12) bereitgestellt wird, der eine Mehrzahl an Kontaktfingern (26, 28) aufweist, die in zumindest einer Reihe angeordnet sind,
eine erste Platine (14) bereitgestellt wird, die zumindest ein elektronisches oder elektrisches Bauelement (16a, 16b) aufweist,
eine zweite Platine (18) bereitgestellt wird, die eine Mehrzahl an durchgehenden Bohrungen (64a-64k) aufweist, die flächig über die zweite Platine (18) zwischen Rändern der zweiten Platine (18) verteilt sind,
ein Kabel (20) bereitgestellt wird, das eine Mehrzahl an Adern (66) aufweist, wobei
die Adern (66) des Kabels (20) in die Bohrungen (64a-64k) der zweiten Platine (18) eingeführt werden, Enden (68a-68k) der Adern (66) etwa im Bereich einer Oberfläche (60) der zweiten Platine (18) angeordnet werden, und wobei die erste Platine (14) an einer Oberfläche (48) mit einer Mehrzahl an punktförmigen ersten elektrischen Kontaktstellen (58a-58k) bereitgestellt wird, deren Flächenverteilung der Flächenverteilung der Enden (68a-68k) der Adern (66) im Bereich der Oberfläche (60) der zweiten Platine (18) entspricht, wobei die Oberfläche (60) der zweiten Platine (18), an der sich die Enden (68a-68k) der Adern (66) befinden, insgesamt im Wesentlichen eben ist, und wobei die erste Platine (14) über die ersten Kontaktstellen (58a-58k) mit den Enden (68a-68k) der Adern (66) elektrisch leitend kontaktiert wird, und wobei
die erste Platine (14) an zumindest einer Schmalseite (44, 50) eine Mehrzahl an zweiten Kontaktstellen aufweist, die als sich in Richtung von der dem Bildsensor (12) zugewandten Oberfläche (40) zur gegenüberliegenden Oberfläche (48) der ersten Platine (14) erstreckende leitfähige Vertiefungen (46, 52) an der zumindest einen Schmalseite (44, 50) ausgebildet sind,
wobei auf die erste Platine (14) eine Vergussmasse (86) aufgebracht wird, wobei der Bildsensor (12) auf die Vergussmasse (86) aufgesetzt und die Kontaktfinger des Bildsensors (12) an den zweiten Kontaktstellen (54, 56) mit der ersten Platine (14) kontaktiert werden.

10. Verfahren nach Anspruch 9, wobei die Adern (66) des Kabels (20) mit ihren Adermänteln (70) in die Bohrungen (64a-64k) eingeführt werden, bis die Adern (66) mit ihren Adermänteln (70) aus den Bohrungen (64a-64k) vorstehen, wobei die Adern (66) in den Bohrungen (64a-64k) mittels Klebstoffs fixiert werden, und wobei entlang der Oberfläche (60) der zweiten Platine (18) senkrecht zu und durch die Adern (66) geschnitten wird, so dass die Enden (68a-68k) der Adern (66) mit Rändern der Bohrungen (64a-64k) an der Oberfläche (60) der zweiten Platine (18) etwa bündig abschließen.

11. Verfahren nach Anspruch 9 oder 10, wobei auf die Enden (68a-68k) der Adern (66) oder auf die ersten Kontaktstellen (58a-58k) leitfähige Fügepunkte aufgebracht werden, und wobei die ersten Kontaktstellen (58a-58k) der ersten Platine (14) auf die Fügepunkte aufgesetzt werden, um die erste Platine (14) mit den Adern (66) zu kontaktieren.

12. Verfahren nach Anspruch 9 oder 10, wobei auf der zweiten Platine im Bereich der Enden der Adern (66) ein flächiges Element angeordnet wird, das aus einem isolierenden komprimierbaren Material gefertigt ist, das partiell mit leitenden Partikeln durchsetzt ist, und wobei die erste Platine (14) mit ihren ersten Kontaktstellen (58a-58k) auf das flächige Element gedrückt wird und an der zweiten Platine (18) festgelegt wird.

13. Verfahren nach einem der Ansprüche 9 bis 12, wobei vor dem Aufbringen der Vergussmasse (86) und vor dem Aufsetzen des Bildsensors (12) an den zweiten Kontaktstellen (54, 56) die Funktion der leitfähigen Verbindungen der ersten und zweiten Platine (14, 18) getestet wird.

## Claims

1. An image pick-up module, particularly for an endoscope, comprising:
an electronic image sensor (12),
a first circuit board (14) which has at least one electronic or electrical component (16a, 16b), the first circuit board (14) being arranged in parallel with a light-receiving surface (22) of the image sensor (12),
a second circuit board (18) which is arranged in parallel with the first circuit board (14) on a side of the first circuit board (14) facing away from the image sensor (12), the second circuit board (18) having a plurality of holes (64a-64k) which are distributed two-dimensionally over the second circuit board (18) between edges of the second circuit board (18), the holes (64a-64k) going through from a surface (60, 62), facing away from the first circuit board (14) to a surface (60, 62) facing the first circuit board (14), of the second circuit board (18),
a cable (20) having a plurality of wires (66) being connected to the second circuit board (18) and leading away from the surface (62), facing away from the first circuit board (14), of the second circuit board (18), the wires (66) being introduced into the holes (64a-64k) of the second circuit board (18), and ends (68a-68k) of the wires being located approximately on the surface (60), facing the first circuit board (14), of the second circuit board (18),
wherein the first circuit board (14) has on a surface (48) facing the second circuit board (18) a plurality of punctiform first electrical contact points (58a-58k), the surface distribution of which corresponds to the surface distribution of the ends (68a-68k) of the wires (66) on the surface (60) of the second circuit board (18), that the surface (60), facing the first circuit board (14), of the second circuit board (18) is essentially level overall, and that the first circuit board (14) is in electrically conductive contact with the ends (68a-68k) of the wires (66) via the first contact points (58a-58k),
**characterized in that** the image sensor has a plurality of contact fingers (26, 28) which are arranged in at least one row and extend perpendicularly to the light-receiving surface (22) of the image sensor towards the first circuit board (14), that the first circuit board (14) has, on at least one narrow side (44, 50), a plurality of second contact points (54, 56), and that the contact fingers (26, 28) of the image sensor (12) are in electrically conductive contact at the second contact points (54, 56), and
that the second contact points (54, 56) are constructed as conductive indentations (46, 52), extending in the direction from the surface (40) facing the image sensor (12) to the opposite surface (48) of the first circuit board (14), on the at least one narrow side (44, 50).

2. The image pick-up module of Claim 1, **characterized in that** the conductive indentations (46, 52) are constructed as essentially half-cut-open cylinders.

3. The image pick-up module of Claim 1 or 2, **characterized in that** the first circuit board (14) is in contact with and connected to the wires (66) at the first contact points (58a-58k) by means of conductive joining points.

4. The image pick-up module of Claim 1 or 2, **characterized in that**, between the first circuit board (14) and the second circuit board (18) in the area of the first contact points (58a-58k), a two-dimensional element is arranged which is manufactured from an insulating compressible material which is interspersed with conductive particles, the two dimensional element being locally compressed at the first contact points (58a-58k) in order to bring the first circuit board (14) electrically conductively into contact with the wires (66).

5. The image pick-up module of any one of Claims 1 through 4, **characterized in that**, on the surface (48), facing the second circuit board (18), of the first circuit board (14), spacers (76) are present which define a narrow gap (74) between the surfaces (48, 60), facing one another, of the first circuit board (14) and of the second circuit board (18).

6. The image pick-up module of any one of Claims 1 through 5, **characterized in that** the wires (66) are fixed in the holes (64a-64k) of the second circuit board (18) by means of adhesive.

7. The image pick-up module of any one of Claims 1 through 6, **characterized in that** the ends (68a-68k) of the wires (66) with their wire cores (69) and their wire jackets (70) end approximately flush with the edges of the holes (64a-64k) on the surface (60) facing the first circuit board (14), of the second circuit board (18).

8. The image pick-up module of any one of Claims 1 through 7, **characterized in that** the image sensor (12) is spaced apart from the first circuit board (14), and that the space between the image sensor (12) and the circuit board (14) is filled with a casting compound (86).

9. A method for producing an image pick-up module, particularly for an endoscope, wherein
an electronic image sensor (12) is provided which has a plurality of contact fingers (26, 28) which are arranged in at least one row,
a first circuit board (14) is provided which has at least one electronic or electrical component (16a, 16b),
a second circuit board (18) is provided which has a plurality of through holes (64a-64k) which are two-dimensionally distributed over the second circuit board (18) between edges of the second circuit board (18),
a cable (20) is provided which has a plurality of wires (66), wherein
the wires (66) of the cable (20) are introduced into the holes (64a-64k) of the second circuit board (18), ends (68a-68k) of the wires (66) are arranged approximately in the area of a surface (60) of the second circuit board (18), and wherein the first circuit board (14) is provided on one surface (48) with a plurality of punctiform first electrical contact points (58a-58k), the surface distribution of which corresponds to the surface distribution of the ends (68a-68k) of the wires (66) in the area of the surface (60) of the second circuit board (18), the surface (60) of the second circuit board (18), on which the ends (68a-68k) of the wires (66) are located, being essentially level overall, and the first circuit board (14) being electrically conductively brought into contact with the ends (68a-68k) of the wires (66) via the first contact points (58a-58k), and wherein the first circuit board (14) has, on at least one narrow side (44, 50), a plurality of second contact points, which are constructed as conductive indentations (46, 52), extending in the direction from the surface (40) facing the image sensor (12) to the opposite surface (48) of the first circuit board (14), on the at least one narrow side (44, 50),
wherein a casting compound (86) is applied to the first circuit board (14), wherein the image sensor (12) is placed onto the casting compound (86) and the contact fingers (26, 28) of the image sensor (12) are brought into contact with the first circuit board (14) at the second contact points (54, 56).

10. The method of Claim 9, wherein the wires (66) of the cable (20) are introduced into the holes (64a-64k) with their wire jackets (70) until the wires (66) with their wire jackets (70) protrude from the holes (64a- 64k), the wires (66) being fixed in the holes (64a-64k) by means of adhesive and cutting being effected along the surface (60) of the second circuit board (18) perpendicularly to and through the wires (66), so that the ends (68a-68k) of the wires (66) end approximately flush with edges of the holes (64a-64k) on the surface (60) of the second circuit board (18).

11. The method of Claim 9 or 10, wherein conductive joining points are applied to the ends (68a-68k) of the wires (66) or onto the first contact points (58a-58k) and wherein the first contact points (58a-58k) of the first circuit board (14) are placed onto the joining points in order to bring the first circuit board (14) into contact with the wires (66).

12. The method of Claim 9 or 10, wherein on the second circuit board in the area of the ends of the wires (66), a two dimensional element is arranged which is manufactured from an insulating compressible material which is partially interspersed with conductive particles and wherein the first circuit board (14) is pressed with its first contact points (58a-58k) onto the two dimensional element and fixed on the second circuit board (18).

13. The method of any one of Claims 9 through 12, wherein, before the casting compound (86) is applied and before the image sensor (12) is placed at the second contact points (54, 56), the functionality of the conductive connections of the first and second circuit board (14, 18) is tested.

## Revendications

1. Module d'enregistrement, en particulier pour un endoscope, comportant :
un capteur d'image électronique (12),
une première platine (14) qui comporte au moins un composant électronique ou électrique (16a, 16b), dans lequel la première platine (14) est disposée parallèlement à une surface réceptrice de lumière (22) du capteur d'image (12),
une seconde platine (18) qui est disposée parallèlement à la première platine (14) sur une face de la première platine (14) opposée au capteur d'image (12), dans lequel la seconde platine (18) comporte une pluralité de perçages (64a - 64k) qui sont traversants d'une surface (60) de la seconde platine (18) opposée à la première platine (14) à une surface (62) de la seconde platine (18) tournée vers la première platine (14) et qui sont répartis sur toute la surface de la seconde platine (18) entre des bords de la seconde platine (18),
un câble (20) qui comporte une pluralité de conducteurs (66), qui est relié à la seconde platine (18) et qui s'éloigne de la surface (62) de la seconde platine (18) opposée à la première platine (14), dans lequel les conducteurs (66) sont introduits dans les perçages (64a - 64k) de la seconde platine (18) et les extrémités (68a - 68k) des conducteurs se trouvent à peu près sur la surface (60) de la seconde platine (18) tournée vers la première platine (14),
dans lequel la première platine (14) comporte sur une surface (48) tournée vers la seconde platine (18) une pluralité de premiers sites de contact électriques en forme de point (58a - 58k) dont la répartition surfacique correspond à la répartition surfacique des extrémités (68a - 68k) des conducteurs (66) sur la surface (60) de la seconde platine (18) de sorte que la surface (60) de la seconde platine (18) tournée vers la première platine (14) soit dans son intégralité sensiblement plane et de sorte que la première platine (14) soit mise en contact électroconducteur par le biais des premiers sites de contact (58a - 58k) avec les extrémités (68a - 68k) des conducteurs (66),
**caractérisé en ce que** le capteur d'image (12) comporte une pluralité de doigts de contact (26, 28) qui sont disposés selon au moins une rangée et qui s'étendent perpendiculairement à la surface réceptrice de lumière (22) du capteur d'image (12) vers la première platine (14), **en ce que** la première platine (14) comporte sur au moins une face étroite (44, 50) une pluralité de seconds sites de contact (54, 56) et **en ce que** les doigts de contact (26, 28) du capteur d'image (12) sont en contact électroconducteur avec les seconds sites de contact (54, 56), et
**en ce que** les seconds sites de contact (54, 56) sont formés en tant qu'évidements conducteurs (46, 52) s'étendant dans la direction allant de la surface (40) tournée vers le capteur d'image (12) à la surface (48) opposée de la première platine (14) sur ladite au moins une face étroite (44, 50).

2. Module d'enregistrement selon la revendication 1, **caractérisé en ce que** les évidements conducteurs (46, 52) sont formés de cylindres sensiblement coupés en leur moitié.

3. Module d'enregistrement selon la revendication 1 ou 2, **caractérisé en ce que** la première platine (14) est mise en contact et reliée avec les conducteurs (66) au niveau des premiers sites de contact (58a - 58k) au moyen de points d'assemblage conducteurs.

4. Module d'enregistrement selon la revendication 1 ou 2, **caractérisé en ce qu'**entre la première platine (14) et la seconde platine (18) dans la zone des premiers sites de contact (58a - 58k) est disposé un élément plan qui est fabriqué dans un matériau isolant comprimable qui est chargé de particules conductrices, dans lequel l'élément plan est comprimé localement au niveau des premiers sites de contact (58a - 58k) afin de contacter électriquement la première platine (14) avec les conducteurs (66).

5. Module d'enregistrement selon l'une quelconque des revendications 1 à 4, **caractérisé en ce que** sur la surface (48) de la première platine (14) tournée vers la seconde platine (18) sont présentes des entretoises (76) qui définissent une fente étroite (74) dans la plage des µm entre les surfaces (48, 60) tournées l'une vers l'autre de la première platine (14) et de la seconde platine (18).

6. Module d'enregistrement selon l'une quelconque des revendications 1 à 5, **caractérisé en ce que** les conducteurs (66) sont fixés dans les perçages (64a - 64k) de la seconde platine (18) au moyen d'une matière adhésive.

7. Module d'enregistrement selon l'une quelconque des revendications précédentes 1 à 6, **caractérisé en ce que** les extrémités (68a - 68k) des conducteurs (66) avec leurs âmes de conducteur (69) et leurs gaines de conducteur (70) rejoignent les bords des perçages (64a-64k) sur la surface (60) de la seconde platine (18) tournée vers la première platine (14) en formant un plan sensiblement identique.

8. Module d'enregistrement selon l'une quelconque des revendications 1 à 7, **caractérisé en ce que** le capteur d'image (12) est écarté de la première platine (14) et **en ce que** l'espace entre le capteur d'image (12) et la platine (14) est rempli avec une masse de remplissage (86).

9. Procédé de fabrication d'un module d'enregistrement, en particulier pour un endoscope, dans lequel
on dispose d'un capteur d'image électronique (12) qui comporte une pluralité de doigts de contact (26, 28) qui sont disposés au moins selon une rangée,
on dispose d'une première platine (14) qui comporte au moins un composant électronique ou électrique (16a, 16b),
on dispose d'une seconde platine (18) qui comporte une pluralité de perçages (64a - 64k) traversants qui sont répartis sur la surface de la seconde platine (18) entre des bords de la seconde platine (18),
on dispose d'un câble (20) qui comporte une pluralité de conducteurs (66),
dans lequel les conducteurs (66) du câble (20) sont introduits dans les perçages (64a - 64k) de la seconde platine (18), des extrémités (68a - 68k) des conducteurs (66) sont disposées dans la zone d'une surface (60) de la seconde platine (18) et dans lequel la première platine (14) est équipée sur une surface (48) d'une pluralité de premiers sites de contact électriques en forme de point (58a - 58k) dont la répartition surfacique correspond à la répartition surfacique des extrémités (68a - 68k) des conducteurs (66) dans la zone de la surface (60) de la seconde platine (18), dans lequel la surface (60) de la seconde platine (18), sur laquelle se trouvent les extrémités (68a - 68k) des conducteurs (66), est dans son intégralité sensiblement plane, et dans lequel la première platine (14) est contactée électriquement par le biais des premiers sites de contact (58a - 58k) avec les extrémités (68a - 68k) des conducteurs (66), et dans lequel
la première platine (14) comporte sur au moins une face étroite (44, 50) une pluralité de seconds sites de contact qui sont formés en tant qu'évidements (46, 52) conducteurs s'étendant dans la direction allant de la surface (40) tournée vers le capteur d'image (12) à la surface opposée (48) de la première platine (14) sur ladite au moins une face étroite (44, 50),
dans lequel une masse de remplissage (86) est appliquée sur la première platine (14), dans lequel le capteur d'image (12) est placé sur la masse de remplissage (86) et les doigts de contact du capteur d'image (12) sont contactés avec la première platine (14) au niveau des seconds sites de contact (54, 56).

10. Procédé selon la revendication 9, dans lequel les conducteurs (66) du câble (20) avec leurs gaines de conducteur (70) sont introduits dans les perçages (64a - 64k) jusqu'à ce que les conducteurs (66) avec leurs gaines de conducteur (70) dépassent des perçages (64a - 64k), dans lequel les conducteurs (66) sont fixés dans les perçages (64a - 64k) au moyen d'une matière adhésive, et dans lequel le long de la surface (60) de la seconde platine (18) les conducteurs (66) sont coupés perpendiculairement aux conducteurs de sorte que les extrémités (68a - 68k) des conducteurs (66) rejoignent les bords des perçages (64a - 64k) sur la surface (60) de la seconde platine (18) en formant un plan sensiblement identique.

11. Procédé selon la revendication 9 ou 10, dans lequel sur les extrémités (68a - 68k) des conducteurs (66) ou sur les premiers sites de contact (58a - 58k) sont appliqués des points d'assemblage conducteurs, et dans lequel les premiers sites de contact (58a - 58k) de la première platine (14) sont placés sur les points d'assemblage afin de contacter la première platine (14) avec les conducteurs (66).

12. Procédé selon la revendication 9 ou 10, dans lequel sur la seconde platine dans la zone des extrémités des conducteurs (66) est disposé un élément plan qui est fabriqué dans un matériau isolant comprimable qui est partiellement chargé de particules conductrices, et dans lequel la première platine (14) avec ses premiers sites de contact (58a - 58k) est pressée sur l'élément plan et fixée sur la seconde platine (18).

13. Procédé selon l'une quelconque des revendications 9 à 12, dans lequel, avant l'application de la masse de remplissage (86) et avant l'installation du capteur d'image (12) sur les seconds sites de contact (54, 56), le fonctionnement des liaisons conductrices des première et seconde platines (14, 18) est vérifié.
